# EUROPEAN PATENT APPLICATION

(11) **EP 1 469 082 A1**
(43) Date of publication of application: **20.10.2004**
(21) Application number: 03008908.0
(22) Date of filing: 16.04.2003
(51) Int. Cl.: C12Q 1/25

(54) **Method of identifying compounds that specifically inhibit the anaphase promoting complex**

(71) Applicant: Boehringer Ingelheim International GmbH, 55216 Ingelheim (DE)
(72) Inventor: VODERMAIER, Hartmut, A-1020 WIEN (AT); GIEFFERS, Christian, A-1020 WIEN (AT); MAURER-STROH, Sebastian, A-1030 WIEN (AT); EISENHABER, Frank, A-1210 WIEN (AT); PETERS, Jan-Michael, A-2100 KORNEUBURG (AT); GMACHL, Michael, A-1140 WIEN (AT)
(74) Representative: Hammann, Heinz, Dr.

(57) **Abstract**

A screening method for identifying specific APC inhibitors comprises a primary screen in which a compound is tested for its ability to interfere with binding of CDH1 or CDC20 to the APC and a secondary screen, in which the compound is tested for its ability to interfere with the activation of the APC by CDH1 or CDC20.

## Description

The present invention relates to the regulation of the cell cycle in eukaryotic cells. In particular, the invention relates to the key regulator of the cell cycle, the Anaphase Promoting Complex (APC) or cyclosome, as a target for chemotherapeutic drugs.

The aim of most chemotherapeutic approaches against cancer is to kill rapidly proliferating cells while leaving non-proliferating, differentiated cells unaffected. Since the state of the components regulating the cell cycle is different between proliferating and quiescent cells, these components are potential targets for anti-cancer drugs.

The Anaphase-Promoting Complex or Cyclosome (APC) is a multisubunit ubiquitin-protein ligase (E3) that controls important transitions in the eukaryotic cell cycle. The main APC targets in mitosis are the anaphase inhibitor securin, whose destruction promotes sister chromatid separation, and mitotic B-type cyclins, which have to be degraded in order to exit from mitosis (reviewed in ^{1,2}) . The APC achieves this by transferring activated ubiquitin residues from ubiquitin-conjugating enzymes (E2 or UBC) onto substrates, resulting in the formation of polyubiquitin chains. These chains act as recognition signal that target the substrate proteins for proteolysis by the 26S proteasome (reviewed in ³).

APC is an unusually complex ubiquitin ligase, sedimenting with an S value of 22 and being comprised of at least 11 core subunits. Sequence motifs found in APC subunits include tetratrico peptide repeats (TPR) in APC3/CDC27, APC6/CDC16, APC7, and APC8/CDC23 ^{4,5}, the DOC domain in APC10/DOC1 ^{6,7} , a cullin homology domain in APC2 ^{8,9}, and a RING finger in APC11 ^{10,11}. Like several other RING finger proteins, APC11 alone is sufficient to catalyze polyubiquitination reactions, although with reduced substrate specificity and regulation ^{10,11}. Furthermore, APC11 tightly interacts with APC2's cullin domain^{12,13}. Similar pairs of cullin and RING proteins are also found at the core of SCF and VHL complex-type ubiquitin ligases, where they form the minimal catalytically active module. These observations imply that APC2 and APC11 constitute the active ubiquitin-conjugating component within holo-APC (the naturally occuring APC), whereas other subunits might confer substrate specificity or play roles in regulation. Alternatively, their main function might be a structural one, as suggested by a 3D model of human APC reconstituted from cryo-electron microscopy. This model shows a cage-like structure that might shield the active subunits on the inside of a reaction chamber¹⁴.

Recently, some evidence for how substrate specificity is achieved has been found. APC activity strictly depends on transient association with one of the activator proteins CDH1 and CDC20, which might be directly involved in substrate recognition (reviewed in ¹⁵). Most recently, APC10 has been implicated in aiding in substrate binding ¹⁶ and processivity ¹⁷ of polyubiquitination. CDH1 and CDC20 possess WD40 repeats, a motif found in other adaptor proteins, and APC10 consists largely of the DOC domain, a conserved motif that is often found in conjunction with other ubiquitination domains and folds into a structure likely to interact with ligands ^{18,19}. Within CDH1, two conserved motifs have been found to be important for interaction with the APC, the N-terminal C-box (also called DRY-box) and the C-terminal amino acid motif isoleucine-arginine (IR) ^{16,20} . Apart from its possible role in substrate recruitment, it is not known how CDH1 activates the APC, or where it binds to the complex.

Reconstitution from recombinant subunits has been crucial for functional and structural studies of several macromolecular complexes, including the SCF ubiquitin ligase, whose crystal structure has recently been solved ²¹. Partially due to its greater complexity, reconstitution of the APC has proven difficult.

The APC has been suggested as a target for chemotherapeutic intervention for the following reasons:
1. The activity of the APC is essential for sister chromatid separation, for the function of the mitotic spindle and for exit from mitosis during cell poliferation. Interfering with this function would prevent tumor cells from completing mitosis.
2. Most tumor cells have highly abnormal karyotypes. They undergo anaphase in the presence of chromosomal damage that would prevent activation of the APC in normal cells. Tumor cells might therefore be especially sensitive to drugs that interfere with APC function.

In WO 96/33286, it is suggested to identify APC inhibitors by incubating the mitotic destruction complex, APC, in a reaction containing a ubiquitin activating enzyme (E1 ), a suitable ubiquitin conjugating enzyme (E2), ubiquitin, ATP and a substrate (e. g. cyclin B) which can be detected either by immunoblotting or because it is labeled radioactively, and determining whether a test compound inhibits the ubiquitination reaction.

WO 98/21326 describes a method for identifying the APC subunits and using these subunits to provide APC in recombinant form to be employed in an assay to identify APC inhibitors.

The screening methods for identifying compounds that inhibit the ubiquitination reaction mediated by the APC, as described in WO 02/00923, are based on the ability of the APC subunit APC11 to autoubiquitinate and to be sufficient to ubiquitinate substrate proteins of native APC.

It was an object of the invention to investigate the mechanism of APC activation in order to provide screening assays for identifying chemical compounds that interfere with this mechanism and thus specifically inhibit the APC.

To solve the problem underlying the invention, in a first step, partial complexes from dissociation of native, purified APC were derived. It was found that APC2 and APC11 can be reversibly dissociated from the complex, leaving an intact residual complex that still interacts with CDH1 and whose activity can be restored by addition of recombinant APC2/11. Furthermore, the inventors isolated a subcomplex consisting of APC1, 2, 4, 5 and 11 that is unable to ubiquitinate substrates or bind CDH1. Thus the role of TPR subunits in CDH1 binding was investigated and it was found that, remarkably, the C-terminal IR interaction motif in CDH1 and CDC20 is shared with APC10 and mediates interaction with the TPR proteins APC3 and APC7. Binding and activation of the APC through recombinant CDH1 was shown to depend on both the C-box and the IR tail *in vitro.* Since these motifs are also found in CDC20, it may be presumed that APC activation by CDC20 occurs through the same mechanism.

Importantly, it was found that peptides bearing the IR (isoleucine-arginine) motif can competitively inhibit APC activation and might prove useful for the development of APC inhibitors and for screening assays to identify APC inhibitors. Based on these findings, new assays for identifying specific APC inhibitors have been developed.

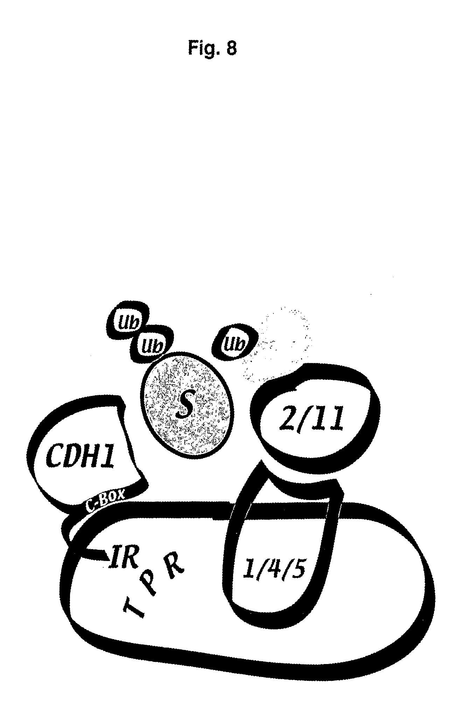

Figure 8 shows a cartoon summarizing the findings on APC assembly and mechanism. APC2/11, which recruit ubiquitin-loaded E2, can be dissociated from the remaining complex. They can also form a stable complex together with APC1/4/5 in the absence of other subunit, especially the TPR proteins. CDH1 interacts with the TPR subunits APC3 and APC7 and might therefore be juxtaposed to APC2/11, consistent with its apparent role as substrate-presenting factor.

The present invention relates to a method for determining whether a compound has the ability to specifically inhibit the APC, wherein
A) in a primary screen a compound is tested for its ability to interfere with binding of CDH1 or CDC20 to the APC by incubating
   a) the APC or one or more APC fragments that are able to bind to CDH1 or CDC20 and
   b) a peptide with the ability to interfere with binding of CDH1 or CDC20 to the APC
   in the presence and in the absence of the test compound, for a period of time sufficient for binding of the peptide b) to the APC (fragment) a), and determining whether the compound competes for the binding, and wherein
B) in a subsequent secondary screen the compound is tested for its ability to interfere with the activation of the APC by CDH1 or CDC20.

An "APC fragment" in the meaning of the present invention may be the full-length APC subunits APC3 or APC7, or variants thereof that have the binding properties of the naturally occuring proteins, or the C-terminal portion of APC3 or APC7 that is responsible for binding to the peptide. In the preferred embodiment, the APC fragment corresponds to the TPR region of the protein, which encompasses amino acids 498 to 824 of human APC3 (accession number NP_001247) or amino acids 297 to 565 of human APC7 (accession number NP_057322), respectively. The APC fragments are preferably employed as recombinant proteins, which can be obtained according to conventional methods by transforming and culturing a suitable host with a plasmid carrying the sequence encoding the protein. The cDNA sequences of the protein components are available from the literature and from databases (see above). The APC subunits or their fragments may contain deviations from the amino acid sequence of the naturally occuring protein as long as these deviations do not impair the protein's functional activity. Preferably, the APC subunits (fragments) are fused to an affinity tag, a protein which is suitable for affinity purification, such as gluthathione S-transferase (GST, Amersham Pharmacia), maltose binding protein (MBP, New England Biolabs) or short tags like His(6) (Qiagen). The preferred host cells are E. coli cells, because they are devoid of endogenous APC. The proteins are purified according to standard protocols, preferably by affinity chromatography.

In the following, the peptide defined in b) is designated a "APC-binding peptide".

In a first aspect, the APC-binding peptide is an "IR peptide". As described above, this peptide has isoleucine-arginine as the C-terminal amino acid motif. In the experiments of the invention, it has been shown that an IR peptide can inhibit binding of the APC to CDH1 or CDC20 and thus activation of the APC. The principle of the method of the invention is to find compounds that compete with the IR peptide and thus function as specific inhibitors for APC activation.

In a preferred emboment, the IR peptide is the C-terminal peptide derived from CDH1 or CDC20.

In an embodiment of the invention, the IR peptide has the sequence CFSKTRSTKESVSVLNLFTRIR (SEQ ID NO:1; authentic human CDH1 C-terminus), or CLRREREKASTSKSSLIHQGIR (SEQ ID NO:2; the authentic human CDC20 C-terminus).

These peptides may be modified with regard to their length and sequence, e.g. they may be shortened at the N-terminus for easier handling, or mutated in view of increasing the binding. These alterations may be carried out routinely, by peptide synthesis, and the candidate IR peptides tested in serial experiments for their binding efficiency.

In another embodiment, the APC-binding peptide is derived from the DRY box, e.g. a peptide with the sequence GDRFIPSR (SEQ ID NO:3), which is derived from human CDH1, or the sequence GDRYIPHR (SEQ ID NO:4) derived from human CDC20. It has been shown that a mutation of this sequence strongly prevents activation of the APC, which is obivously a result of reduced binding of the mutated CDH1 to the APC.

In the method of the invention, the ability of the test compound to interfere with binding of CDH1 or CDC20 to the APC can be tested according to various assay principles.

In a first embodiment, the primary screening assay (step A) is a fluorescence polarization assay (FPA).

The fluorescence polarization assays that can be employed in the method of the invention are well known in the art. The basic principle of fluorescence polarization (FP) involves a change in the rotational Brownian motion of a small molecule upon binding to a larger molecule. Thus, following a binding event, a small molecule that previously tumbled rapidly in solution, will assume the slower motion of the large molecule to which it has bound. The change in the mobility of a small fluorescent ligand can be detected with high sensitivity by measuring the depolarization of the light emitted following excitation with polarized light. FP permits direct measurement of complex formation in solution without its separation from reactants.

Thus, in a first embodiment, the primary screen (step A) comprises the steps of
a) incubating, in the presence and absence of a test compound, a mixture comprising the APC or an APC fragment and a fluorescence labeled APC-binding peptide for a period of time sufficient for binding,
b) determining binding of the fluorescence-labeled APC-binding peptide to the APC (fragment) by measuring fluorescence polarization in the label; and
c) comparing the fluorescence polarization values obtained in the presence and absence of the test compound, wherein a reduction in the value is indicative of the compound's ability to interfere with binding of the APC-binding peptide to the APC.

Suitable fluorescent labels are commercially available, examples are fluorescein, rhodamine, oregon green, Bodipi FL, etc.; methods for conjugation are well known in the art. The fluorescent value must be detectable at low concentrations.

In a preferred embodiment, holo APC, preferably from human cells, is used in this type of FP assay.

In alternative embodiment of step A, the extent of binding is determined by measuring the physical proximity of the binding partners upon incubation, e.g. by means of a FRET assay (fluorescence resonance energy transfer, as described by ²⁷ or by ²⁸). FRET can only be achieved if certain conditions are fullfilled, i.e. fluorophor pairs with overlapping emission and excitation wavelenghts, like europium/allophycocyanin, europium/Cy5, europium/PE (all commercially available from Wallac, Turku, Finnland) and an minimal proximity of these fluorophors below 5-10nM.ln a FRET assay, the APC-binding peptide and an APC fragment carry fluorophors that form a suitable FRET pair. These fluorophors may be present on the molecule by being bound to antibodies directed against the affinity label present on the APC subunit (or a fragment thereof) as obtained after purification, e.g. GST or the myc epitope, or they are directly coupled to the APC subunit (or fragment). The APC-binding peptide usually carries the fluorophor either directly (direct coupling is usually achieved by commercial custom service, e.g. Wallac) or the biotinylated peptide is used in combination with fluorophor-labeled streptavidin. When coupled to antibodies, the fluorophors are added to the reaction after its completion. No further washing steps are necessary and signals (excitation at 340 nm and emission measurement at 665 nm in the case of the FRET pair allophycocyanin and Europium) are measured, e.g. after incubation at 4ºC for 30 min, allowing the binding of the antibodies and the subsequent energy transfer between the fluorophors.

If a compound is able to interfere with binding, it reduces the FRET signal.

Thus, in this embodiment, the primary screen of step A comprises the steps of
a) incubating, in the presence and absence of a test compound, a mixture comprising an APC-binding peptide and an APC fragment,
   i) wherein the binding partners carrying fluorophors to form a fluorescence resonance energy transfer pair are incubated for a period of time sufficient for binding, or
   ii) wherein the binding partners are incubated for a period of time sufficient for binding and subsequently provided with reagents carrying fluorophors tho form a fluorescence resonance energy transfer pair,
b) measuring the FRET signal and comparing the FRET signal obtained in the presence and absence of the test compound, wherein a reduction in the signal is indicative of the compound's ability to interfere with binding of the APC-binding peptide to the APC fragment.

As a specificity control, unlabeled peptide that competes for binding with the labeled peptid, is present in excess.

In an alternative embodiment, the primary screen (step A) in the method of the invention is based on an ELISA-type assay employing reagents that recognize the binding partners.

In an embodiment, such reagents are antibodies; the assay is an ELISA-type assay that may be carried out according to standard methodology that is commercially available. By way of example, such an assay may be conducted as follows: One of the binding partners (APC subunit or a fragment thereof) is immobilized on a microtiter plate by means of an antibody (against the APC subunit or a fragment thereof or against a tag, e.g. GST, His, Myc) that is bound to the plate. The other binding partner, the APC-binding peptide, is added in biotinylated form, in the presence and absence of the test compound, and binding is determined by addition of streptavidin (or avidin) that carries a detectable label, e.g. horseradish peroxidase, alkaline phosphatase or a fluorophor.

This assay may also be conducted in a reverse manner: in this case the biotinylated APC-binding peptide is immobilized on a streptavidin plate and the APC subunit (fragment) is added in solution. Binding is determined, in the presence or absence of the test compound, by addition of a labeled antibody against the APC subunit (or against a tag) or by addition of a labeled secondary antibody that binds to an antibody against the APC subunit or against a tag.

In both variants, a test compound with the ability to inhibit binding of the APC-binding peptide to the APC subunit is identified by a reduced signal. For specificity control, non-biotinylated APC-binding peptide is present in excess.

Alternatively, the primary screening assay of step A may be a radioactive assay, e.g. a scintillation proximity assay (SPA).

The APC subunit (fragment) is immobilized on a solid support, e.g. an SPA or LEAD Seeker bead (Amersham), using an epitope tag such as GST or His. The APC-binding peptide carrying a radioactive label, e.g. ³³P, ³H, ¹²⁵I, is added in the absence or presence of test compounds, incubated for a period of time sufficient for binding and the amount of radioactivity remaining associated with the beads is measured.

In an alternative embodiment, the method of step A is carried out as a Amplified Luminescent Proximity Homogeneous Assay (ALPHA).

This so-called AlphaScreen, which is commercially available (Life Sciences Perkin Elmer) is a bead-based non-radioactive assay. When a biological interaction brings the beads together, a cascade of chemical reactions acts to produce a greatly amplified signal. On laser excitation, a photosensitizer in the "donor" bead converts ambient oxygen to a more excited singlet state. The singlet state oxygen molecules diffuse across to react with a thioxene derivative in the "acceptor" bead generating chemiluminescence at 370 nm that further activates fluorophors contained in the same bead. The fluorophors subsequently emit light at 520-620 nm.

In the absence of a specific biological interaction, the singlet state oxygen molecules produced by the donor bead go un-detected without the close proximity of the acceptor bead. As a result, only a very low background signal is produced.

When biological interactions bring the donor and acceptor beads into close proximity, a highly amplified signal with output in the 520-620 nm range is generated.

The application of this assay in the method of the present invention may be as follows: the biotinylated APC-binding peptide is bound to the streptavidin-coated donor beads and the APC subunit (fragment) is bound to the acceptor beads via a tag on the protein and a corresponding binding partner on the beads, e.g. a GST tag and an anti-GST antibody, a His6 tag and NiNTA (Ni-nitrilotriacetic acid), a Myc tag and an anti-Myc antibody, a HA tag (hemagglutinin) an anti-HA antibody or a Flag tag and anti Flag antibodies. The donor and acceptor beads are incubated for a period of time sufficient for binding of the APC-binding peptide and the APC fragment, in the presence or absence of the test compound, and the chemiluminescence signals are compared. A reduction of the signal is indicative of the compound's ability to interfere with binding.

To confirm that a compound that has been identified in the above-described primary screens of step A) according to its ability to interfere with binding of an APC-binding peptide to the APC (fragment) will also inhibit holo APC and is therefore a drug candidate which is capable of inhibiting the cell's entry into the subsequent cell cycle, the compound is, in the next step B), subjected to a secondary screen employing holo APC.

Together with the ubiquitin-activating enzyme E1 and ubiquitin-conjugating enzymes (E2s) the APC assembles multiubiquitin chains on a variety of regulatory proteins and thus targets them for proteolysis by the 26S proteasome. To initiate sister chromatid separation, the APC has to ubiquitinate the anaphase inhibitor securin, whereas exit from mitosis requires the APC-mediated ubiquitination of B-type cyclins. These reactions depend on activation of the APC by CDC20 and CDH1, which transiently associate with the APC at the end of mitosis and in G1, respectively. APC substrates contain either a destruction (D) or a KEN box sequence whose presence is required for recognition by the APC (3).

In the secondary screen of step B), an APC with the ability of the native APC to ubiquitinate APC substrate protein in a strictly regulated manner (the regulation depending inter alia, on binding to regulatory proteins like CDH1 or CDC20 and on the phosphorylation status) is employed, e.g. immunoprecipitated APC, preferably from human cells, in particular from HeLa cells. Since a candidate compound useful for therapy should interfere with mitotic APC in order to inhibit cell cycle progression, the APC employed in the this step is preferably mitotic APC. Such mitotic APC can be obtained according to standard methods, e.g. from Nocodazole-arrested HeLa cells, as described in ²⁶.

Alternatively, recombinant APC may be used, which can be produced according to the method described in WO 98/21326. This APC may be immobilized on a solid support, preferably beads, covalently associated with antibodies against an APC subunit, e.g. APC3/CDC27; in the case of recombinant APC, it may be present in solution.

In addition to the mitotic APC, either immunoprecipitated on beads or in solution, the following components are employed in the reaction, which occurs in the presence or absence of the candidate compound: E1, E2, in particular UBCH5b or UBCH10, or a combination of both, ubiquitin, either radioactively labeled for direct detection of polyubiquitin chains or unlabeled for detection of substrate conjugates, an ATP regenerating system and substrate proteins such as CyclinB or Securin, preferably from human source. Both substrate proteins may be tagged at the carboxy-terminus with a myc-epitope for antibody detection and a his(6) tag for affinity purification, or alternatively with a GST tag. The substrate proteins are either labeled with ¹²⁵I or detectable through a carboxy-terminal tag, e.g. the myc tag recognized by the 9E10 antibody. The secondary screening assay system also requires the presence of an APC activator, i.e. CDH1 or CDC20 in recombinant form, preferably expressed in insect cells. Reactions can either be analysed by SDS-PAGE following phosphorimaging (when using iodinated proteins) or Western blotting using appropriate antibodies (9E10, anti ubiquitin).

Alternatively, this assay can also be performed on microtiter plates covered with antibodies, e.g. against GST. Substrate proteins like CyclinB or Securin, when fused to GST, can be captured on the plates after the reaction is completed. The amount of associated ubiquitin can be measured either directly, when using ¹²⁵I-ubiquitin or quantified using antibodies against ubiquitin, or against a tag associated with ubiquitin. These antibodies are either directly labeled, i.e. with a fluorophor or with ¹²⁵l, or will be detected with a secondary, labeled antibody.

A suitable ubiquitin activating enzyme is the wheat UBA1 E1 (gene bank accession number M55604), however, UBA1 E1 from other species may also be used, which can be purified on a ubiquitin affinity matrix according to published procedures (e.g. ^{28,29}).

CDH1 or CDC20, respectively, are employed as recombinant proteins, preferably expressed in insect cells, as described in ²³.

An example for an ubiquitin conjugating enzyme (E2) is, in a preferred embodiment, the human variant UBCH5b (gene bank accession number U39317), although, also in this case, UBCH5b homologs from other species, e.g. Xenopus laevis, may be employed. Alternatively, UBCH5a or UBCH5c can be used. Preferably the ubiquitin conjugating enzyme E2 is fused to an affinity tag.

To provide ATP for the ubiquitination reaction, a so-called "ATP regenerating system" is employed (³¹).

Untagged ubiquitin is commercially available (Sigma). Recombinantly produced ubiquitin is fused to different tags for purification, i.e. His(6), GST or for detection, i.e. myc-epitope, HA-epitope. In both cases, ubiquitin comprises the N-terminal 76 amino acids required for its function.

To make sure that the secondary assay of step B) is indeed suitable to confirm that the compounds inhibit APC activation, a control reaction is peformed prior to the screen. In such control reaction the APC activators are omitted and the background activity of the APC determined.

Compounds identified in the screening method of the invention, which function as specific APC inhibitors, are expected to arrest cells in metaphase of mitosis; this arrest is expected to subsequently induce apoptotic cell death.

Due to this ability, such compounds are drug candidates for the therapy of cancer.

To test the ability of a compound to inhibit tumor cell proliferation, primary human tumor cells are incubated with the compound identified in the screen and the inhibition of tumor cell proliferation is tested by conventional methods, e.g. bromo-desoxy-uridine or ³H incorporation. Compounds that exhibit an antiproliferative effect in these assays may be further tested in tumor animal models and used for the therapy of tumors.

Toxicity and therapeutic efficacy of the compounds identified as drug candidates can be determined by standard pharmaceutical procedures, which include conducting cell culture and animal experiments to determine the IC₅₀, LD₅₀, ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using one or more physologically active carriers and excipients. Methods for making such formulations can be found in manuals, e.g. "Remington Pharmaceutical Sciences".

In another aspect, the present invention relates to peptidomimetics that are derived from the APC binding peptides, i.e. CFSKTRSTKESVSVLNLFTRIR (SEQ ID NO:1), CLRREREKASTSKSSLIHQGIR (SEQ ID NO:2), GDRFIPSR (SEQ ID NO:3) or GDRYIPHR (SEQ ID NO:4). Based on the amino acid sequence, peptidomimetics can be obtained by known methods, e.g. as described by ³², or by ³³. These peptidomimetics may be tested for their ability to inhibit APC activation, as described above for the secondary screening assay, and to inhibit tumor proliferation and used as cancer therapeutics like small molecule compounds.

### Brief description of the figures:

### Figure 1

APC2 and APC11 can be dissociated from the APC
A) Silver-stained gel (left) and western blot (right) of APC re-immunoprecipitated after treatment with different pH conditions.
B) CDH1 binding assay. Holo-APC and Δ2/11-APC were incubated with buffer or recombinant CHD1 and probed with CDH1 antibodies for the presence of endogenous (lower band) and recombinant (upper band, shifted due to HisHA-tag) CDH1.
C) Western blot of an *in vitro* ubiquitination assay with holo-APC and Δ2/11-APC, using recombinant securin as a substrate.

### Figure 2

Reconstitution of active, CDH1-responsive holo-APC from Δ2/11-APC and recombinant APC2 and APC11
A) *In vitro* ubiquitination assay with purified GST-APC11/APC2 subcomplexes and securin in the presence of either UBC4 or UBCx.
B) Western blot of Δ2/11-APC bound to αAPC4 beads after incubation with APC2/11 lysates.
C) *In vitro* ubiquitination assay with reconstituted APC and UBCx. The substrate securin is detected by western blot.
*D) In vitro* ubiquitination assay with reconstituted APC in the absence and presence of CDH1.

### Figure 3

A subcomplex consisting of APC1, 2, 4, 5, 11 elutes separately from holo-APC from an anion exchange resin.
A) HeLa cell lysates were subjected to Resource Q anione exchange chromatography and eluted with a KCI gradient. αAPC4-IPs from different fractions were separated by SDS-PAGE and visualized by silver staining (left) and western blotting (right).
B) *In vitro* ubiquitination assay with holo-APC or Q₃₅₀-APC and recombinant securin as substrate. Substrate and ubiquitin chains were detected by western blot.
C) CDH1 binding assay. IPs of holo-APC or Q₃₅₀-APC where incubated with recombinant HisHA-tagged CDH1 and immunoblotted for the presence of endogenous and recombinant CDH1.

### Figure 4

A possible APC-coactivator interaction module

3D model of the TPR domain of APC3, based on predicted structural homologies to PEX5. Individual TPRs are depicted in different colors. The PTS1 peptide crystallized with PEX5 has been replaced by the C-terminal pentapeptide of APC10 (YRSIR), which is known to interact with APC3.

### Figure 5

The TPR-containing subunits APC3 and APC7 mediate APC interactions with C-terminal IR motifs
A) Matrix-coupled C-terminal peptides were incubated with insect cell lysates containing recombinant APC subunits. 10% of input (In), supernatant (S), and 100% of bound material (B) were analyzed by western blot.
B) Late-eluting Q column fractions containing mainly TPR subunits were used as input in peptide binding assays as in A), with CDC20/CDH1 peptides or two different control peptides.
C) Fractions containing holo-APC were used as input in the described peptide binding assay.
D) Peptide binding assay with recombinantly expressed fragments of APC7.

### Figure 6

C-terminal CDH1/CDC20 peptides containing the IR motif inhibit CDH1 binding and APC activation
A) CDH1 binding assay. APC-IPs were incubated with recombinant CDH1 in the presence or absence of different peptides. CDH1 binding was analyzed by quantitative western blotting with [¹²⁵l]-labelled secondary antibodies.
B) *In vitro* ubiquitination assay. APC-IPs treated as in A were washed and used in ubiquitination assays with [¹²⁵l]-labelled cyclin B as substrate.

### Figure 7

Binding and activation of the APC depends on CDH1's C-terminal IR motif and the C-box
A) CDH1 binding assay. αAPC3-lPs from APC-containing fractions or APC-free control fractions were incubated with insect cell lysates containing recombinant wild-type CDH1 or CDH1 mutated in the C-box (ΔCB), the IR motif (ΔIR), or in both motifs (2xΔ).
B) *In vitro* ubiquitination assay. APC was incubated with wild-type or mutant CDH1 as in A and used in a ubiquitination assay with recombinant securin as substrate.

### Figure 8

Cartoon summarizing our current views of APC assembly and mechanism APC2/11, who recruit ubiquitin-loaded E2, can be dissociated from the remaining complex. They can also form a stable complex together with APC1/4/5 in the absence of other subunit, especially the TPR proteins. CDH1 interacts with the TPR subunits APC3 and APC7 and might therefore be juxtaposed to APC2/11, consistent with its apparent role as substrate-presenting factor.

### Figure 9

### Δ2/11-APC sediments as an intact complex

Sucrose density gradient centrifugation of holo-APC (A) and Δ2/11-APC (B). Fractions were concentrated adnd anlayzed by western blot with APC antibodies. As input sample and antibody control, holo-APC was used in A and B.

### Figure 10

Anion exchange chromatography separates distinct populations of APC subunits

Hela extracts were separated on a Source15Q column and eluted with a KCI gradient. Protein-containing fractions were collected and analyzed by western blotting for APC subunits, four of which are shown as examples.

In the Examples, if not otherwise stated, the following materials and methods were used:

### a) Antibodies, peptides, mutagenesis and recombinant protein expression

Antibodies raised against APC2, APC3/CDC27, APC8/CDC23, CDH1, APC10 and CDC26 have been described ^{7,10,14,25,26} . Antibodies against APC4, APC5, APC6/CDC16, APC7 and APC11 were raised by immunizing rabbits with synthetic peptides coupled to Keyhole Limpet Hemocyanin (APC4: CIVIKVEKLDPELDS, SEQ ID NO:5. APC5: CELTSRDEGERKMEKEEL; SEQ ID NO:6. APC6: CLETSRKTPDSRPSL; SEQ ID NO:7. APC7: CQKMEKEESPTDATQEED; SEQ ID NO:8. APC11: CRQEWKFKE, SEQ ID NO:9). UBCx antibodies were raised in mice against recombinantly expresses UBCx.

Synthetic peptides used in binding and inhibition assays were CLRREREKASTSKSSLIHQGIR (CDC20 C-terminus; SEQ ID NO:2), CFSKTRSTKESVSVLNLFTRIR (CDH1 C-terminus;SEQ ID NO:1), CESDLHSLLQLDAPIPNAPPARW (CDC20 N-terminus; SEQ ID NO:10), and CAVWSLSSCKPGFGVD (control;SEQ ID NO:11).

Expression and purfication of recombinant E1, E2 enzymes as well as His-myc-tagged securin was performed as described ¹⁰.

Recombinant HisHA-tagged CDH1 was expressed and purified as described ²³. The QuickChange Site-directed mutagenesis kit (Stratagene, La Jolla, CA) was used to generated the DRY box deletion (GDRFIPSR to AAAAAAAA; SEQ ID NO:12). For truncation of the C-terminal IR, a premature stop codon was introduced by PCR.

Baculoviruses encoding the CDH1 mutants, His-APC2, HisHA-tagged APC3, 5, 6, 7, 8 and HA-APC11 were generated using the BAC-TO-BAC system (Invitrogen), and proteins were expressed in Sf9 or Hi5 cells.

### b) Isolation and Reconstitution of Δ2/11-APC

Extracts from logarithmically growing HeLa cells were prepared in buffer A (20 mM Tris-HCl, pH 7.5, 100 mM NaCl, 0.2% NP-40, 20 mM - glycerophosphate, 10% glycerol, 1 mM NaF, 0.5 mM DTT), and APC was immunopurfied using an APC3 peptide antibody ¹⁴ . Bead-bound APC was washed for 90 min with buffer B (50 mM NaP04, 500 mM NaCl, 1 % Triton X-100, 1 mM DTT, pH 6.5) for removal of APC2 and APC11 followed by a brief 5 min wash with buffer C (20 mM Tris/HCl, 150 mM NaCl, 0.02% Tween-20, 0.5 mM DTT, pH 7.6). Complexes were eluted with buffer C plus 1 mg/ml CDC27-peptide for 2 h, and holo-APC removed from the eluate by immunoprecipitation with APC2-30 monoclonal antibodies ²⁵. The remaining supernatant containing the purified Δ2/11-APC was finally re-immunoprecipitated using APC4 antibodies or analyzed directly by western blotting or sucrose density gradient centrifugation.

For reconstitution, Δ2/11-APC on APC4 antibody beads was incubated for 30 min at 4°C in extracts prepared from Sf9 insect cells (in buffer A). That had been infected with baculoviruses encoding His-tagged APC2, HA-tagged APC11 or both proteins. Extracts were centrifuged at 100.000xg for 60 min before incubation with Δ2/11 APC. Beads were subsequently washed 5x with buffer C and analysed for APC2/11 binding and ubiquitination activity.

### c) CDH1 binding and in vitro ubiquitination assays

Immunopurified holo-APC or subcomplexes were washed and incubated with purified recombinant CDH1 (0.5-1.0 µg per 10 µl bead volume) in TBS/T, or with insect cell lysates in TBS/T containing recombinant CDH1 proteins as indicated. Peptides for competition were used at 1 mg/ml, with the addition of 0.5 mM DTT to the buffer. Subsequently, bead-bound complexes were washed 3x with TBS/T and immunoblotted for binding of CDH1 or used in ubiquitination assays. In vitro ubiquitination reactions were performed as described²⁶. Securin-myc-his substrate was detected by immunoblotting with monoclonal anti-Myc 9E10 antibodies. Alternatively, an iodinated N-terminal recombinant fragment of sea urchin cyclin B (amino acids 13-110) was used and visualized by autoradiography¹⁴.

### d) Anion exchange chromatography

Pellets from 5x10⁹ logarithmically grown HeLa cells (4C, Belgium) were lysed in 20 mM TrisCl pH7.7, 150 mM KCI, 0.5 mM DTT, 0.1% Tween-20, Protease Inhibitor mix (Sigma) using a french press and cleared by 100,000xg centrifugation for 1 h. After filtration through a 0.45 µm filter, extracts were loaded onto a 60ml Source15Q anion exchange column (Amersham) at 4 ml/min. The column was washed with 20 mM Tris-Cl pH7.7, 150 mM KCI, 0.5 mM DTT until a drop in A₂₈₀ indicated the end of the flowthrough. Elution was carried out with a gradient from 150 mM to 600 mM KCl. Elution fractions were collected, desalted using PD10 gravity flow colums (Pharmacia) and concentrated in Centriprep cartridges.

### e) Peptide binding assays

Peptide interaction assays were carried out as described in Wendt et al, 2001¹⁸. Synthetic peptides (see above) were immobilized via N-terminal cysteine residues onto maleinimide-activated POROS matrix (Applied Biosciences) in 0.1 M HEPES, pH 7.9 for 3 hours. After washing with slightly acidic buffers, 100 µg of the resulting matrix were incubated with insect cell lysates containing recombinant APC subunits, or with fractions from anion chromatography, in total volumes of 100 µl of TBS/T pH 7.5. After stringent washing in TBS/T 0.5 M NaCl, samples were analyzed by SDS-PAGE and immunoblotted with 12CA5 anti-HA antibodies or antibodies against APC subunits.

### Example 1

The catalytic subunits APC2 and APC11 can be dissociated from holo-APC

Total or partial reconstitution from recombinant subunits has been essential for understanding the assembly and function of several macromolecular complexes. However, to date all attempts to reconstitute the APC have failed, and little information about the function of each of its at least 11 subunits is available. So far, the only recombinant subcomplex that has yielded any functional information is APC2/11, consisting of the cullin domain protein APC2 and the small RING-H2 finger protein APC11 ^{12,13}. This subcomplex is able to form polyubiquitin chains and transfer some of them onto APC substrates, but in a non-regulated manner. To gain further insight into the functions and molecular interactions of APC subunits, a reverse strategy was pursued and conditions that dissociate individual subunits from holo-APC searched for.

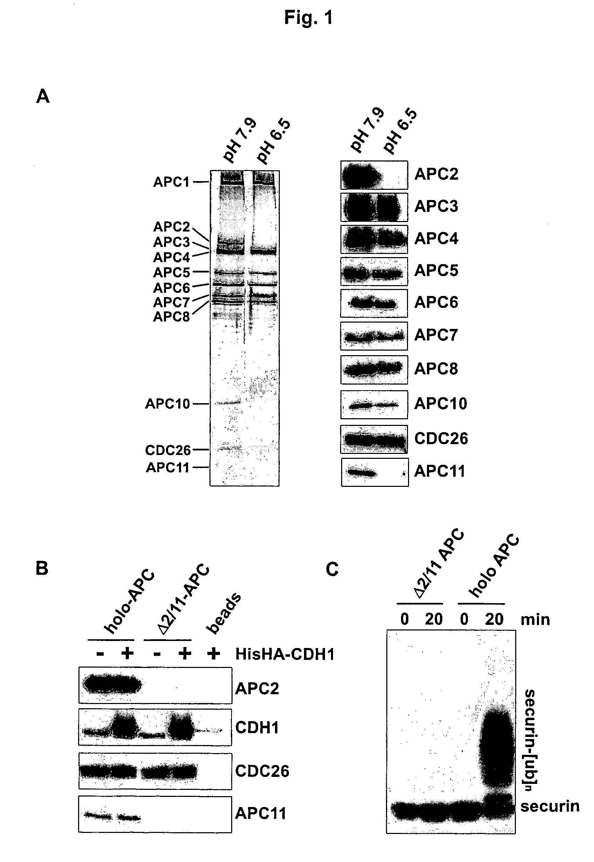

Based on previously developed methods to immunopurify human APC¹⁴, APC bound to APC3 antibody beads was treated with various buffer conditions. Surprisingly, it was found that slightly lowered pH reduced the amount of APC2 and APC11 bound to holo-APC. After extensive washing of immunopurified APC with a high salt sodium phosphate buffer of pH 6.5 (see material and methods for details) and subsequent peptide elution, complexes still containing APC2 were depleted by immunoprecipitation with APC2 antibodies. Re-immunoprecipitation with APC4 antibodies showed that the remaining complex (henceforth called Δ2/11-APC) has indeed quantitatively lost APC2 and APC11 (Figure 1A). This pH-dependent dissociation can first be observed at pH 7.0 and increases with further lowering of the pH. Decrease below pH 6.3 caused considerable loss of APC from the antibody beads, and it was not possible to assay loss of additional subunits under these conditions. The only other subunit slightly affected by lowered pH appears to be APC10, whereas the relative amounts of all other subunits were unchanged (Figure 1 A).

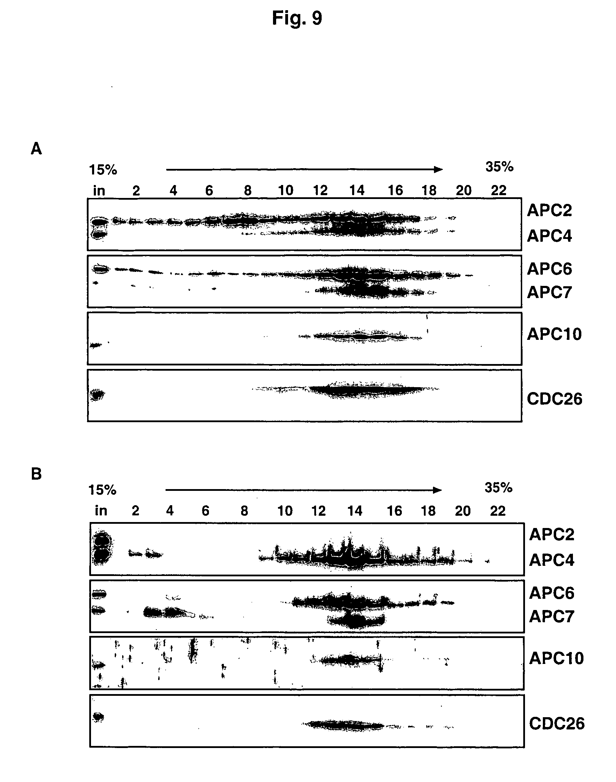

As further confirmation that Δ2/11-APC is still an intact macromolecular complex, it was fractionated by sucrose density gradient centrifugation. All remaining subunits of Δ2/11-APC sedimented as a single peak of about 22S (Figure 9), this S value being almost identical to the one observed with holo-APC ¹⁴ . This indicates that the loss of APC2/11 does not grossly change mass, shape and molecular composition of the APC.

Having confirmed the integrity of Δ2/11-APC, the inventors wanted to know which functional properties of native APC it still retains. Interaction with activators of the WD40 repeat family is essential for substrate-specific APC activity. Δ2/11-APC was tested for binding to the activator CDH1. Both holo-APC and Δ2/11-APC contained substoichiometric amounts of bound endogenous CDH1, and incubation with purified recombinant CDH1 resulted in additional loading of more activator (Figure 1B). APC2 and APC11 therefore appear to be dispensable for CDH1 binding, consistent with the observations that CDH1 can neither bind to nor activate recombinant APC2/11 complexes (data not shown). Subsequently CDH1-loaded holo-APC and Δ2/11-APC were analyzed for ubiquitination activity towards the substrate securin. Whereas holo-APC converts securin into ubiquitin conjugates of higher molecular mass in the presence of ATP, E1 and E2 enzymes, Δ2/11-APC is completely inactive in this *in vitro* ubiquitination assay (Figure 1 C). APC2/11 are therefore not only sufficient, but also strictly required for ubiquitination activity of the APC.

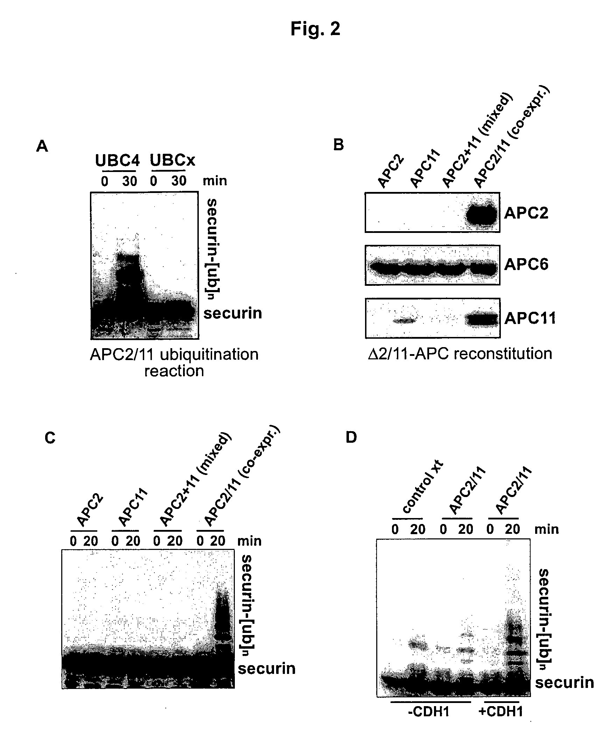

Despite the apparent integrity of Δ2/11-APC (Figure 1 A and Figure 9), the possibility could not be fully excluded that the low pH treatment during preparation might have caused irreversible damage to the subcomplex, and that this damage has lead to loss of enzymatic activity. It was hence tried to reconstitute active holo-APC by loading Δ2/11 -APC with recombinant APC2/11 complexes generated in baculovirus-infected insect cells. As shown in Figure 2B, APC2/11 did indeed bind back to the depleted complex. To distinguish a functional interaction from unspecific binding, an *in vitro* ubiquitination reaction with UBCx as E2 enzyme was carried out. According to these experiments, APC2/11 is only supported by the E2 enzyme UBC4, whereas holo-APC is active in the presence of either UBCx or UBC4 (Figure 2A). Any substrate ubiquitination observed with UBCx must therefore be due to holo-APC activity. Δ2/11-APC reconstituted with co-expressed APC2/11 has regained UBCx-dependent activity towards securin (Figure 2C), whereas incubation with APC11 or APC2 alone is not sufficient (Figure 2C). Interestingly, re-binding was only observed when coexpressed APC2 and APC11 was used - neither protein did bind alone, and posttranslationally mixed APC2 and APC11 also failed to bind and reconstitute activity (Figure 2B). This is consistent with the observation of Tang and coworkers ¹². That APC2 and APC11 only interact upon co-expression, and implies that this interaction either creates a common interface for APC association or is essential for proper folding and structural integrity of these two subunits.

It was tested if reconstituted APC would be responsive to CDH1 activation. Indeed, ubiquitination activity of reconstituted APC in the absence of CDH1 was only slightly increased compared to Δ2/11-APC incubated with control extracts, but significant activation was achieved in the presence of CDH1 (Figure 2D). This strict CDH1-dependence is a hallmark of holo-APC and further proof that the observed activity is not due to APC2/11 subcomplexes, since they are not CDH1-responsive (data not shown and ¹²).

### Example 2

APC1, 2, 4, 5 and 11 form a stable subcomplex

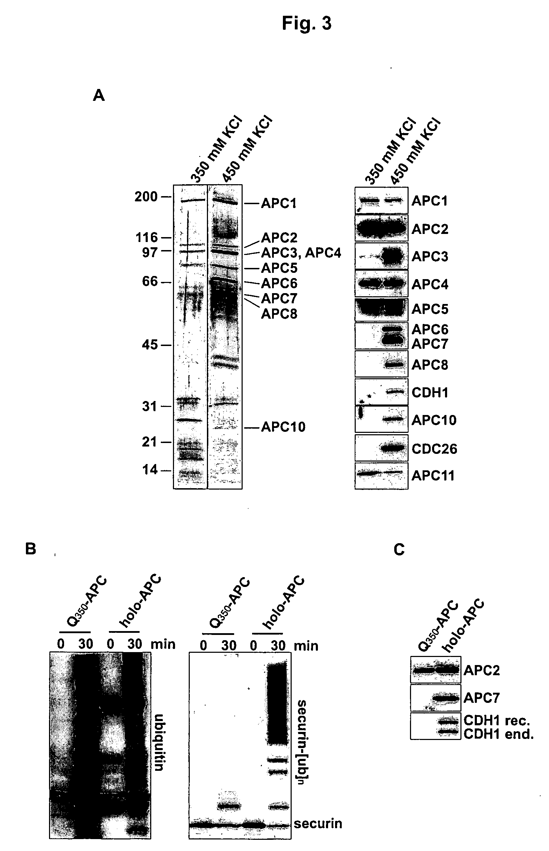

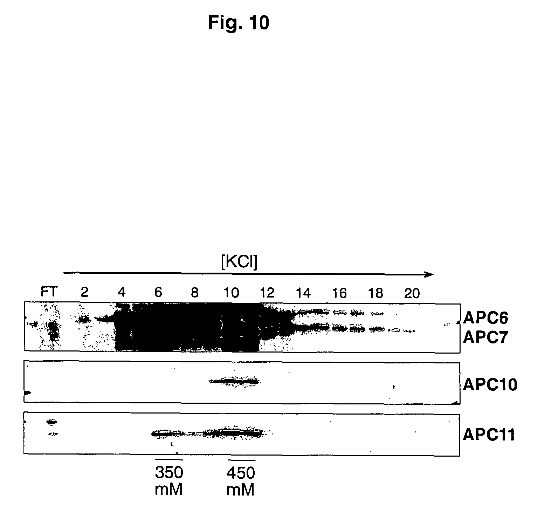

In previous sucrose density gradient or anion exchange fractionation experiments, it had been noted that APC2 is not only found in one peak fraction with holo-APC, but sometimes also in a distinct second peak whose sedimentation behavior implied association with additional proteins (data not shown and Figure 9). To investigate this further, extracts from logarithmically growing HeLa cells were subjected to anion exchange chromatography on a Source15Q column. Bound material was eluted with a potassium chloride gradient. Holo-APC (identified by the presence of all 11 known subunits) eluted in a sharp peak at around 450 mM KCI (Figure 10). While some subunits, especially the TPR-containing proteins APC3, APC6 and APC7, were additionally found trailing through several more fractions, other subunits formed a distinct second peak eluting already at 350 mM KCI (Figure 10). These fractions did indeed contain APC2 as well as its close interaction partner, APC11. To test if these subunits are actually associated in an independent subcomplex, APC3-containing complexes were depleted from the 350 mM fractions using APC3 antibodies. Subsequent immunoprecipitation with antibodies against APC2, APC4 or APC5 reproducibly yielded the same set of APC1, 2, 4, 5, and 11 in apparently stoichiometric amounts (Figure 3A), without significant contamination by any other subunit. This subcomplex, hereafter called "Q₃₅₀-APC", was stable even after peptide elution from the APC4 antibody and subsequent re-precipitation with APC5 antibodies (data not shown).

It could not be judged if Q₃₅₀-APC was generated during extract preparation or fractionation, or if it represents a physiological subcomplex, either as an assembly intermediate or even a functional entity. Nevertheless, its apparent stability opened the opportunity to investigate its properties further. In a ubiquitination assay, Q₃₅₀ -APC did not show significant activity towards securin (Figure 3B), despite the presence of APC2 and APC11. This is unexpected given the ability of recombinant APC2/11 complexes to ubiquitinate APC substrates, but might support a model in which additional subunits would limit unregulated access of substrates to the catalytically active subunits. Consistent with this, Q₃₅₀-APC was still able to generate polyubiquitin chains (data not shown). This implies that the deficiency in securin ubiquitination might be a direct consequence of the absence of substrate specificity factors. Therefore Q₃₅₀-APC and APC from the 450mM KCI fractions were incubated with recombinant CDH1 and assayed binding. As shown in Figure 3C, holo-APC was associated with endogenous and additional recombinant CDH1. Neither form of CDH1, on the other hand, was bound to Q₃₅₀-APC (Figure 3C). The lack of substrate ubiquitination in the absence of CDH1 is therefore consistent with a role for CDH1 in substrate recognition, as implied by studies from several labs.

### Example 3

TPR domains and a C-terminal peptide motif as APC-CDH1 interaction modules

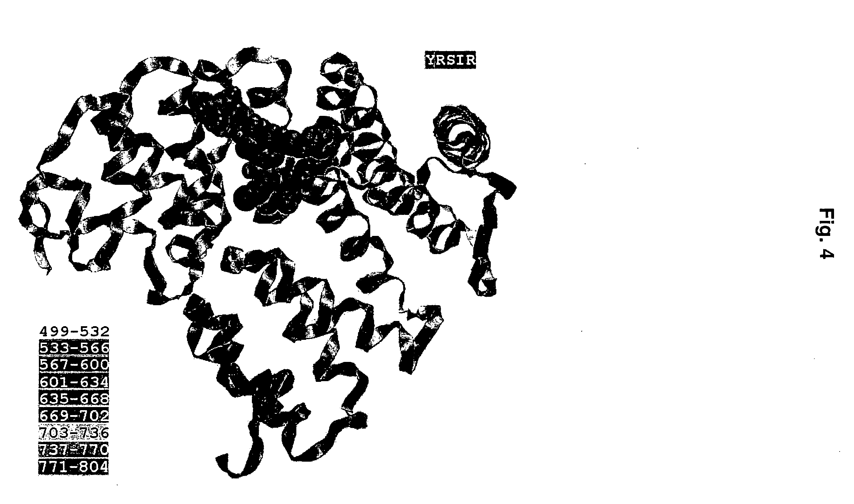

To understand APC activation, it is important to map the molecular interactions between CDH1 and APC subunits. Several determinants in CDH1 have been found to influence APC binding, namely the so-called C-box ²⁰, the C-terminal isoleucine-arginine (IR) motif ^{16,20}, and phosphorylation status of CDH1^{22,23}. On the other hand, which subunits and sequences within the APC are responsible for interaction with CDH1 is unclear. The analysis of Δ2/11-APC and Q₃₅₀-APC has revealed that APC2/11 are dispensable for CDH1 binding and that APC1, 2, 4, 5 and 11 are not sufficient for CDH1 binding. This leaves APC3, APC6, APC7, APC8, CDC26 and APC10 as possible CDH1 receptors. Interestingly, APC3, 6, 7 and 8 all contain multiple TPRs, a motif present in several adaptor proteins like the co-chaperone Hop or the peroxisomal importing receptor PEX5, where it is involved in interactions with C-terminal peptides. Structure prediction tools suggest that the TPR domains in APC3, APC6 and APC7 fold in a very similar manner to PEX5 (Figure 4), and the inventors have previously shown that APC3 binds APC10 through APC10's extended C-terminal peptide ¹⁸. Figure 4 shows APC3's TPR domain modeled on the PEX5 structure ²⁴, with PEX5's cognate PTS1 peptide replaced by APC10's C-terminal pentapeptide, YRSIR. Strikingly, it was noted that APC10 orthologues from different species all share this conserved IR (LR in yeast) dipeptide with the APC activators CDH1 and CDC20. This remarkable conservation in several proteins in the APC context suggested an important, possibly common functional role for this motif.

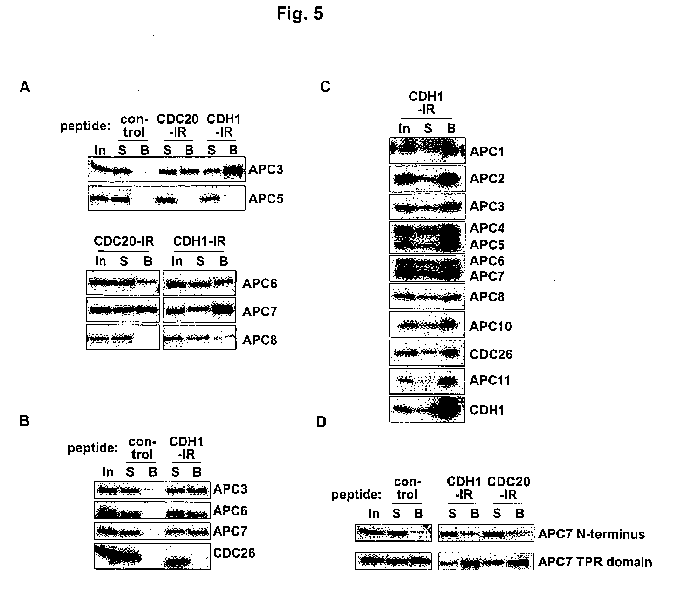

To test if APC3 would also interact with the C-termini of CDH1 and its related activator CDC20, insect cell lysates containing recombinantly expressed APC3 were incubated with synthetic peptides coupled to a matrix through their N-terminus. After washing, peptide-bound material was analyzed by western blot. In this assay, about 10% of input APC3 bound to the C-terminal peptide of CDC20, and even stronger binding was observed to a CDH1 C-terminal peptide (Figure 5A). The interaction was specific, since APC3 did not bind to a control peptide, and recombinant APC5 interacted with neither the CDC20 nor the CDH1 peptide (Figure 5A). When the other three TPR subunits were tested, it was found that APC8 did not bind either peptide, and APC6 bound only in low amounts that were also observed to bind to control peptides (Figure 5A and data not shown). APC7, on the other hand, interacted strongly with the CDH1 C-terminus and moderately with CDC20's C-terminus (Figure 5A), in a manner very similar to APC3. Interestingly, APC3 and APC7 are highly related in their primary sequences. In budding yeast, which lacks APC7, they share Cdc27p as their closest homologue. This suggests that APC3 and APC7 might have originated from gene duplication and might still have overlapping functions.

In order to exclude any non-specific effects caused by incorrect folding of recombinantly expressed proteins, an alternative source of APC subunits was used in the peptide binding assays. As described above, late-eluting fractions from the ReSource Q column contained considerable amounts of the TPR subunits APC3, APC6, and APC7, while other subunits were found in lower abundance (Figure 10). Although these TPR subunits can still be partially co-precipitated, they do not seem to be in stoichiometric complexes anymore (data not shown), and the inventors thus considered them a satisfactory source of "native" APC subunits. As with recombinant proteins, APC3 and APC7 were significantly retained by the CDH1 peptide matrix, whereas APC6 was not (Figure 5B). CDC26, which is also present in these fractions, showed no binding at all (Figure 5B).

It was tested if CDH1's C-terminus would be sufficient to interact with holo-APC. For this, APC-containing fractions were incubated with the peptide matrix and analyzed for binding of APC by detection of all subunits. Every known subunit was strongly enriched on the CDH1 peptide matrix (Figure 5C) but not on control peptides (data not shown). Since APC6 and CDC26 from late-eluting fractions did not bind the CDH1 peptide by themselves (Figure 5B), their interaction, as well as that of other subunits like APC2 and APC11, must be mediated through the holo-complex. Surprisingly, endogenous CDH1 was also enriched on the peptide matrix (Figure 5C). Since purified APC is loaded with substoichiometric amounts of endogenous CDH1 (Figures 1 B and 3C), this co-binding could be mediated by dimerization of APC. An alternative and more intuitive explanation is the presence of more than one receptor for IR peptides per complex (see discussion).

Lastly, the region in APC7 responsible for binding to IR peptides was mapped. An APC7 fragment containing the N-terminal 297 amino acids did not exhibit binding, whereas the C-terminal part (amino acids 298 to 565) comprising the block of TPRs was stronlgy enriched on the peptide matrix (Figure 5D). This shows that the TPR domain is sufficient for interaction with IR peptides.

### Example 4

C-terminal peptides of CDH1 and CDC20 inhibit APC activation

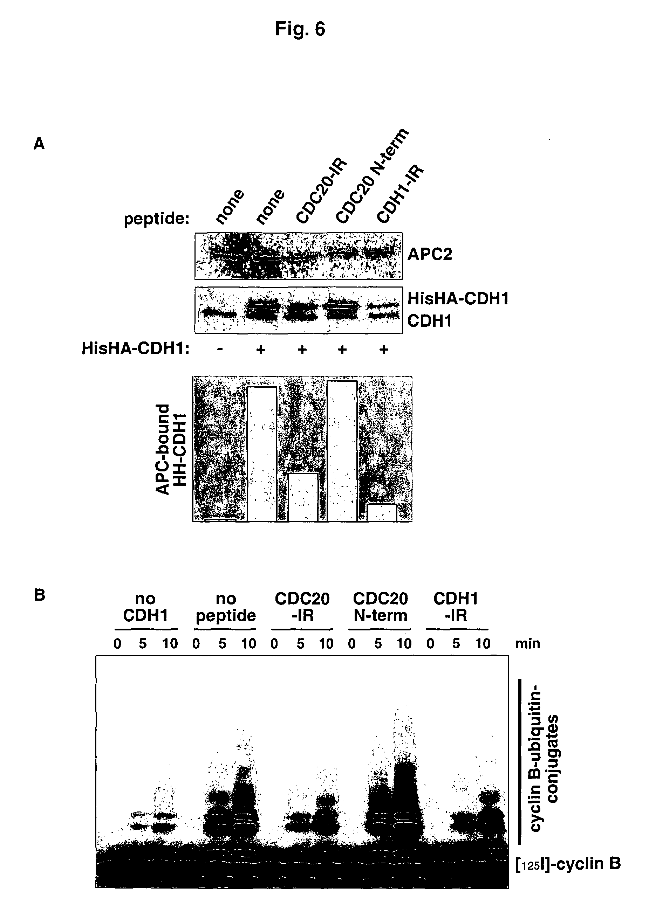

What are the mechanistic consequences of the observed interactions between IR tail peptides and APC subunits? To address this, it was tested if the peptides would influence APC activity in *in vitro* ubiquitination assays. Immunopurified APC was loaded with recombinant CDH1 in the presence or absence of IR-tail or control peptides, washed, and used to ubiquitinate a radioactively labeled fragment of cyclin B (Figure 6B). The C-terminal peptides of CDH1 and CDC20 both blocked APC activation efficiently, lowering ubiquitination activity to almost the level of APC that has not been activated by exogenous CDH1. In contrast, an N-terminal CDC20 peptide had no effect on APC activation. Quantitative immunoblotting using radiolabeled antibodies against APC2 and CDH1 showed that the inhibition corresponded with diminished binding of CDH1 (Figure 6A). As in the peptide binding assays (Figure 5), CDH1's C-terminal peptide had a stronger effect than the one from CDC20. This competitive inhibition of CDH1 loading implies that the IR tails do not function in catalysis, but are rather responsible for actively targeting CDH1 to the APC.

### Example 5

CDH1 binding and APC activation depends on two motifs in CDH1

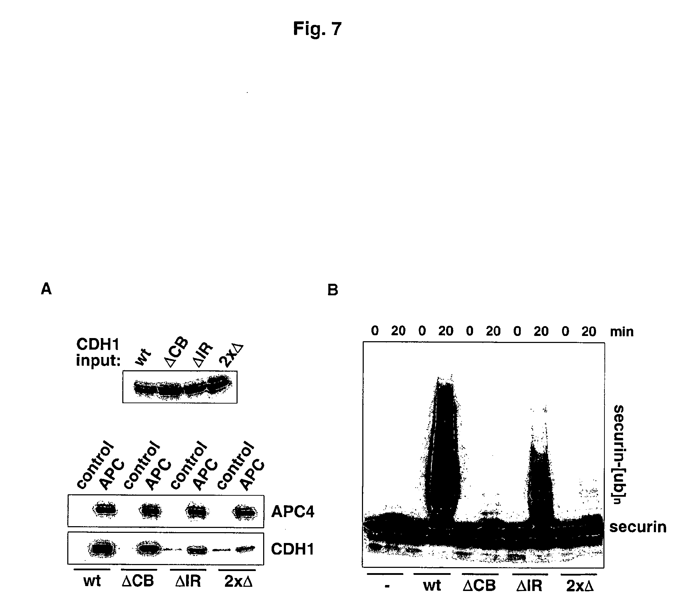

Having established that C-terminal IR tails are sufficient to interact with APC and interfere with its activation, the inventors wanted to know how their loss would influence CDH1 activity. To this end, a baculovirus encoding CDH1 with a deletion of the C-terminal dipeptide (ΔIR) was generated. Another conserved motif located in CDH1's N-terminal region, the C-box, has been shown to be required for APC binding in yeast ²⁰. Therefore CDH1 versions with mutations in the C-box (ΔDRY), and in both motifs (2xΔ) were also generated. After expression in insect cells, the lysates were incubated with antibody-bound APC or control antibody beads, washed, and tested for activity and binding of recombinant CDH1. Figure 7A shows that both mutations in the DRY box and in the IR tail diminished binding significantly but not completely. Combining both mutations resulted in a further decrease in affinity, indicating that DRY box and IR tails cooperate to achieve optimal APC binding (Figure 7A).

Similar effects were seen in ubiquitination assays: the IR tail deletion mutant retained significant levels of activity, whereas the DRY box mutant was almost completely inactive (Figure 7B). Taken together, these data strongly argue for additive roles of CDH1's conserved APC interaction motifs, and they also show that binding and activation go hand in hand.

### References

1. Peters, J. M. The anaphase-promoting complex: proteolysis in mitosis and beyond. *Mol Cell* **9,** 931-43. (2002).
2. Harper, J. W., Burton, J. L. & Solomon, M. J. The anaphase-promoting complex: it's not just for mitosis any more. *Genes Dev* **16,** 2179-206. (2002).
3. Pickart, C. M. Mechanisms underlying ubiquitination. *Annu Rev Biochem* **70,** 503-33. (2001).
4. Lamb, J. R., Michaud, W. A., Sikorski, R. S. & Hieter, P. A. Cdc16p, Cdc23p and Cdc27p form a complex essential for mitosis. *Embo J* **13**, 4321-8. (1994).
5. Blatch, G. L. & Lassle, M. The tetratricopeptide repeat: a structural motif mediating protein-protein interactions. *Bioessays* **21**, 932-9. (1999).
6. Kominami, K., Seth-Smith, H. & Toda, T. Apc10 and Ste9/Srw1, two regulators of the APC-cyclosome, as well as the CDK inhibitor Rum1 are required for G1 cell-cycle arrest in fission yeast. *Embo J* **17**, 5388-99. (1998).
7. Grossberger, R. et al. Characterization of the DOC1/APC10 subunit of the yeast and the human anaphase-promoting complex. *J Biol Chem* **274**, 14500-7. (1999).
8. Zachariae, W. et al. Mass spectrometric analysis of the anaphase-promoting complex from yeast: identification of a subunit related to cullins. *Science* **279**, 1216-9. (1998).
9. Yu, H. et al. Identification of a cullin homology region in a subunit of the anaphase-promoting complex. *Science* **279**, 1219-22. (1998).
10. Gmachl, M., Gieffers, C., Podtelejnikov, A. V., Mann, M. & Peters, J. M. The RING-H2 finger protein APC11 and the E2 enzyme UBC4 are sufficient to ubiquitinate substrates of the anaphase-promoting complex. *Proc Natl Acad Sci U S A* **97**, 8973-8. (2000).
11. Leverson, J. D. et al. The APC11 RING-H2 finger mediates E2-dependent ubiquitination. *Mol Biol Cell* **11**, 2315-25. (2000).
12. Tang, Z. et al. APC2 Cullin protein and APC11 RING protein comprise the minimal ubiquitin ligase module of the anaphase-promoting complex. *Mol Biol Cell* **12,** 3839-51. (2001).
13. Ohta, T., Michel, J. J., Schottelius, A. J. & Xiong, Y. ROC1, a homolog of APC11, represents a family of cullin partners with an associated ubiquitin ligase activity. *Mol Cell* **3***,* 535-41. (1999).
14. Gieffers, C., Dube, P., Harris, J. R., Stark, H. & Peters, J. M. Three-dimensional structure of the anaphase-promoting complex. *Mol Cell* **7,** 907-13. (2001).
15. Vodermaier, H. C. Cell cycle: Waiters serving the Destruction machinery. *Curr Biol* **11**, R834-7. (2001).
16. Passmore, L. A. et al. Doc1 mediates the activity of the anaphase-promoting complex by contributing to substrate recognition. *Embo J* **22**, 786-96. (2003).
17. Carroll, C. W. & Morgan, D. O. The Doc1 subunit is a processivity factor for the anaphase-promoting complex. *Nat Cell Biol* **4**, 880-7. (2002).
18. Wendt, K. S. et al. Crystal structure of the APC10/DOC1 subunit of the human anaphase-promoting complex. *Nat Struct Biol* **8**, 784-8. (2001).
19. Au, S. W., Leng, X., Harper, J. W. & Barford, D. Implications for the ubiquitination reaction of the anaphase-promoting complex from the crystal structure of the Doc1/Apc10 subunit. *J Mol Biol* **316,** 955-68. (2002).
20. Schwab, M., Neutzner, M., Mocker, D. & Seufert, W. Yeast Hct1 recognizes the mitotic cyclin Clb2 and other substrates of the ubiquitin ligase APC. *Embo J* **20**, 5165-75. (2001).
21. Zheng, N. et al. Structure of the Cul1-Rbx1-Skp1-F boxSkp2 SCF ubiquitin ligase complex. *Nature* **416,** 703-9. (2002).
22. Zachariae, W., Schwab, M., Nasmyth, K. & Seufert, W. Control of cyclin ubiquitination by CDK-regulated binding of Hct1 to the anaphase promoting complex. *Science* **282**, 1721-4. (1998).
23. Kramer, E. R., Scheuringer, N., Podtelejnikov, A. V., Mann, M. & Peters, J. M. Mitotic regulation of the APC activator proteins CDC20 and CDH1. *Mol Biol Cell* **11**, 1555-69. (2000).
24. Gatto, G. J., Jr., Geisbrecht, B. V., Gould, S. J. & Berg, J. M. Peroxisomal targeting signal-1 recognition by the TPR domains of human PEX5. *Nat Struct Biol* **7**, 1091-5. (2000).
25. Gieffers, C., Peters, B. H., Kramer, E. R., Dotti, C. G. & Peters, J. M. Expression of the CDH1-associated form of the anaphase-promoting complex in postmitotic neurons. *Proc Natl Acad Sci U S A* **96**, 11317-22. (1999).
26. Kramer, E. R., Gieffers, C., Holzl, G., Hengstschlager, M. & Peters, J. M. Activation of the human anaphase-promoting complex by proteins of the CDC20/Fizzy family. *Curr Biol* **8***,* 1207-10. (1998).
27. Gershkovich, A.A. and Kholodovych, V.V. (1996), J Biochem Biophys Meth 33, 135
28. Matayoshi, E.D. (1990), *Science* **247**, 954
29. Hatfield P.M., Callis J. & Vierstra R.D. (1990) *J Biol Chem* **265**, 15813-7
30. Hatfield P.M. & Vierstra R.D. (1992)*Biol Chem* **267**, 14799-803
31. Murray, A. (1991) Methods Cell Biol. 36, 581-605
32. Kieber-Emmons T, Murali R, Greene MI. Therapeutic peptides and peptidomimetics. Curr Opin Biotechnol 1997 Aug;8(4):435-41
33. Ripka A.S., and Rich D.H., Peptidomimetic design. Curr Opin Chem Biol 1998 Aug; 2(4):441-52

## Claims

1. A method for determining whether a test compound has the ability to specifically inhibit the APC, wherein
A) in a primary screening step, a compound is tested for its ability to interfere with binding of CDH1 or CDC20 to the APC by incubating
a) the APC or one or more APC fragments that are able to bind to CDH1 or CDC20 and
b) a peptide with the ability to interfere with binding of CDH1 or CDC20 to the APC
in the presence and in the absence of the test compound, for a period of time sufficient for binding of the peptide b) to the APC (fragment) a), and determining whether the compound competes for the binding, and wherein
B) in a subsequent secondary screening step the compound is tested for its ability to interfere with the activation of the APC by CDH1 or CDC20.

2. The method of claim 1, wherein the APC fragment b) is APC3 or APC7 or a fragment thereof.

3. The method of claim 1, wherein the peptide b) is the C-terminal peptide derived from CDH1.

4. The method of claim 3, wherein the peptide a) has the amino acid sequence as shown in SEQ ID NO:1.

5. The method of claim 1, wherein the peptide b) is the C-terminal peptide derived from CDC20.

6. The method of claim 5, wherein the peptide a) has the amino acid sequence as shown in SEQ ID NO:2.

7. The method of claim 1, wherein the primary screening step A) comprises the steps of
a) incubating, in the presence and absence of a test compound, a mixture comprising the APC or an APC fragment and a fluorescence-labeled APC-binding peptide for a period of time sufficient for binding,
(b) determining binding of the fluorescence-labeled APC-binding peptide to the APC (fragment) by measuring fluorescence polarization in the label; and
(c) comparing the fluorescence polarization values obtained in the presence and absence of the test compound, wherein a reduction in the the value is indicative of the compound's ability to inhibit binding of the APC-binding peptide to the APC.

8. The method of claim 7, wherein the mixture in step a) contains holo APC.

9. The method of claim 1, wherein the primary screening step A) comprises the steps of
a) incubating, in the presence and absence of a test compound, a mixture comprising an APC-binding peptide and an APC fragment,
i) wherein the APC-binding peptide and the APC fragment that carry fluorophors forming a fluorescence resonance energy transfer (FRET) pair are incubated for a period of time sufficient for binding, or
ii) wherein the APC-binding peptide and an APC fragment are incubated for a period of time sufficient for binding and subsequently provided with reagents carrying fluorophors forming a fluorescence resonance energy transfer pair,
b) measuring the FRET signal and comparing the FRET signal obtained in the absence and in the presence of the test compound, wherein a reduction in the signal is indicative of the compound's ability to interfere with binding of the APC-binding peptide to the APC fragment.


SEQUENCE LISTING

<110> Boehringer Ingelheim International GmbH

<120> Method for identifying compounds that specifically
      inhibit the Anaphase Promoting Complex

<130> Case 14/081

<140>
<141>

<160> 12

<170> PatentIn Ver. 2.1

<210> 1
<211> 22
<212> PRT
<213> Homo Sapiens

<400> 1
Cys Phe Ser Lys Thr Arg Ser Thr Lys Glu Ser Val Ser Val Leu Asn
  1               5                  10                  15

Leu Phe Thr Arg Ile Arg
             20

<210> 2
<211> 22
<212> PRT
<213> Homo Sapiens

<400> 2
Cys Leu Arg Arg Glu Arg Glu Lys Ala Ser Thr Ser Lys Ser Ser Leu
  1               5                  10                  15

Ile His Gln Gly Ile Arg
             20

<210> 3
<211> 8
<212> PRT
<213> Homo Sapiens

<400> 3
Gly Asp Arg Phe Ile Pro Ser Arg
  1               5

<210> 4
<211> 8
<212> PRT
<213> Homo Sapiens

<400> 4
Gly Asp Arg Tyr Ile Pro His Arg
  1               5

<210> 5
<211> 15
<212> PRT
<213> Homo Sapiens

<400> 5
Cys Ile Val Ile Lys Val Glu Lys Leu Asp Pro Glu Leu Asp Ser
1               5                   10                  15

<210> 6
<211> 18
<212> PRT
<213> Homo Sapiens

<400> 6
Cys Glu Leu Thr Ser Arg Asp Glu Gly Glu Arg Lys Met Glu Lys Glu
1               5                   10                  15

Glu Leu

<210> 7
<211> 15
<212> PRT
<213> Homo Sapiens

<400> 7
Cys Leu Glu Thr Ser Arg Lys Thr Pro Asp Ser Arg Pro Ser Leu
1               5                   10                  15

<210> 8
<211> 18
<212> PRT
<213> Homo Sapiens

<400> 8
Cys Gln Lys Met Glu Lys Glu Glu Ser Pro Thr Asp Ala Thr Gln Glu
1               5                   10                  15

Glu Asp

<210> 9
<211> 9
<212> PRT
<213> Homo Sapiens

<400> 9
Cys Arg Gln Glu Trp Lys Phe Lys Glu
1               5

<210> 10
<211> 23
<212> PRT

<213> Homo Sapiens

<400> 10
Cys Glu Ser Asp Leu His Ser Leu Leu Gln Leu Asp Ala Pro Ile Pro
1               5                   10                  15

Asn Ala Pro Pro Ala Arg Trp
            20

<210> 11
<211> 16
<212> PRT
<213> Artificial Sequence

<220>
<223> Description of Artificial Sequence: control
      peptide

<400> 11
Cys Ala Val Trp Ser Leu Ser Ser Cys Lys Pro Gly Phe Gly Val Asp
1               5                   10                  15

<210> 12
<211> 8
<212> PRT
<213> Artificial Sequence

<220>
<223> Description of Artificial Sequence: mutation

<400> 12
Ala Ala Ala Ala Ala Ala Ala Ala
1               5